# EUROPEAN PATENT APPLICATION

(11) **EP 1 808 071 A1**
(43) Date of publication of application: **18.07.2007**
(21) Application number: 05781914.6
(22) Date of filing: 12.09.2005
(51) Int. Cl.: A01K 67/027, A01K 67/00, A61B 6/00, A61D 7/00, G01T 1/161

(54) **CONSTRUCTION OF ARTERIAL OCCLUSIVE DISEASE ANIMAL MODEL**

(30) Priority: 13.09.2004 JP 2004265671; 13.09.2004 JP 2004265677
(71) Applicant: Japan Health Sciences Foundation, Tokyo 103-0001 (JP)
(72) Inventor: IIDA, Hidehiro, c/o Nat. Cardiovascular Centre, Suita-shi, Osaka 5658565 (JP); OHTA, Yoichiro, Takatsuki-shi, Osaka 5691041 (JP); TERAMOTO, Noboru, c/o Nat. Cardiovascular Centre, Suita-shi, Osaka 5658565 (JP); HAYASHI, Takuya, c/o Nat. Cardiovascular Centre, Suita-shi, Osaka 5658565 (JP)
(74) Representative: Stoner, Gerard Patrick
(86) International application number: PCT/JP2005/016756
(87) International publication number: WO 2006/030737

(57) **Abstract**

The present invention relates to a method for generating a non-human model animal of an arterial occlusive disease, wherein the method is characterized in that an artery is occluded at a specific site by ligating it or with an autologous blood clot. In particular, the present invention relates to a method and a kit for generating a cardiac infarction model, as well as a cardiac infarction animal model obtained by employing the method or kit, characterized in that in a non-human experimental animal, a blood vessel is ligated downstream of an arterial site of the heart to be occluded, followed by occluding the artery at said site. In addition, the present invention also relates to a method and a system for generating a non-human animal model of arterial embolus, as well as an arterial embolus animal model obtained by employing the method or system, characterized by adding an coagulating agent to autologous blood to form an autologous blood clot and subsequently employing an angiographic apparatus in delivering the autologous blood clot into an artery.

## Description

### TECHNICAL FIELD

The present invention relates to a method and a kit or a system for generating an animal model of an arterial occlusive disease, as well as an arterial embolus animal model obtained by the method or system. Specifically, the present invention relates to a method and a kit or a system for generating a non-human model animal of an arterial occlusive disease, as well as an arterial embolus animal model obtained by the method or system, characterized by occluding an artery at a specific site by ligating it or with an autologous blood clot.

### BACKGROUND ART

In the past, a number of models of heart diseases such as cardiac infarction have been used in medical studies, for example, of the circulatory system and in organ transplantation, or alternatively in the development of medicaments for circulatory system diseases. Recently, these models of heart diseases also have become utilized in studies on regenerative medicine.

Regarding conventional models of cardiac infarction, as methods for generating cardiac infarction models, for example, ligature of coronary artery with thoracotomy is commonly employed, but conventional ligature causes fibrillation directly after the procedure and has an extremely great likelihood of leading to death. For these reasons, there are relatively often used methods for generating models which employ ameroid rings (means by which a casein component which has been formed into a ring-like shape expands by absorbing body fluid and gradually occludes a blood vessel in two weeks or so). Major problems of these procedures are that occlusion is at varied degrees, non-occluded or incompletely occluded cases also occur, and so they fail to provide stable models of pathological conditions. In addition, a high mortality within a relatively short period (for example, one month after the procedure) is also problematic.

Therefore, there has been a need for the development of cardiac infarction models avoiding disadvantages as described above, in medical studies of the circulatory system and on organ transplantation, or alternatively in the development of medicaments for circulatory system diseases, and additionally in studies on regenerative medicine.

Arterial embolus leads to various diseases and disorders, but it cannot yet be said that methods for treating these diseases and disorders have been established. Typical examples of diseases and disorders caused by arterial embolus are cardiac infarction and cerebral accident. Cerebral accident ranks high in the causes of death in developed countries, including Japan. Especially with respect to therapeutic treatments in its acute stages, agents such as thrombolytics and nueroprotectives are expected to be effective, but many of these agents have failed in clinical trials and a very small number of agents have been found to be effective. This is true for other diseases and disorders caused by arterial embolus. As a cause, it is believed that there is a large "species difference" in efficacy of drugs, since conventional developments of medicaments use rodent animals such as rats and mice. Thus, it has become desirable that in primate animals which are animals closely related to humans, and medium- and large-sized mammals, models of pathological conditions which are closer to clinically pathological conditions are constructed and evaluated. Up to now, several models of ischemia have been generated employing medium- and large-sized animals, such as monkeys, cats, dogs, and pigs, and while these models display some aspects of clinically pathological conditions as methodology, there are few models which are believed to correspond to actual clinical situations. Especially for pathological conditions in which occlusion occurs in intracranial arteries, such as the middle cerebral artery, internal carotid artery, vertebral artery, and basilar artery (acute cerebral trunk arterial occlusion syndrome), among acute cerebral ischemia, there is a strong desire for effective therapeutic strategies with few complications, and an urgent need of basic investigations with animal models of these diseases, since their prognosis is poor (with a mortality of 10 to 15%) and their onset occurs frequently in young and middle-aged people.

Conventional methods for generating animal models of arterial occlusion, in particular, of cerebral trunk arterial occlusion, are divided largely into two approaches: vessel-pressing and intravascular embolus. The former is a procedure in which a trunk artery of interest is blocked by means of a clip, ligature, or the like, with an invasive method such as craniotomy, orbital enucleation (see, for example, Hudgins, WR, et. al., Stroke, 1970 Mar-Apr; 1(2): 107-11.), or others, and as a typical example is a procedure by which a horizontal portion of the middle cerebral artery is closed. Although models using cerebral trunk arterial occlusion are conventionally known as representative models of ischemia in medium- and large-sized animals, many such models have disordered sites that are limited to the basal nucleus of the brain and there are few models which can give rise to damage in the cortex of the brain, that is most discussed as a site causing cerebral function disorders in clinical situations. In fact, it is also known that in behavioral evaluation of these animal models, permanent paralysis appears at extremely low levels. On the other hand, as approaches using vascular embolus, models are also known in which blood vessels are occluded intravascularly with plastics, silicones, blood clots, or the like (see Watanabe, O., et al., Stroke, 8: 61-70, 1975; and Kito, G, et. al., J Neurosci Methods, 2001 Jan 30; 105(1): 45-53.). However, these models lack procedures for exact injection into intracranial arteries of interest and identification and for evaluating ischemia, and thus result in variations in the distribution of cerebral ischemic portions and pathological conditions, making it difficult to conduct selective and quantitative experiments.

As mentioned above, conventional vascular embolus procedures suffer from disadvantages of having a low certainty and varied degrees of vascular embolus and ischemia after the formation of the vascular embolus, and lacking in quantitiveness. In addition, these procedures are highly invasive to animals and have a disadvantage of causing great damage to animals. If animal models can be obtained which overcome these disadvantages, it would be likely that such models would promote researches on diseases and disorders caused by arterial embolus, including stroke, and also lead to the discovery of new approaches for treating these diseases and disorders. In addition, animal models which overcome these disadvantages would be very useful in developing new medicaments for treatment or prophylaxis of diseases and disorders caused by arterial embolus, and in conducting researches on regenerative medicine.

### DISCLOSURE OF THE INVENTION

It is an object to be attained by the present invention to develop a method for generating a cardiac infarction model which has a small variation in the degree of occlusion and a low level of animal mortality and provides stable pathological conditions. Furthermore, it is another object to be attained by the present invention to develop a method for generating a vascular embolus animal model which has a high certainty of vascular embolus formation and subsequent ischemia and a small variation in their degree and is capable of selective and quantitative analysis. Also in the present invention, it is a problem to be solved by the present invention to develop a method for generating a vascular embolus animal model which gives low invasiveness and causes little damage to animals.

The present inventors have made extensive researches, taking the above-described problems into account, with the result that it has been found that by occluding an artery at a specific site by ligating it or with an autologous blood clot, the above-described problems can be solved, whereby the completion of the invention has been achieved.
Specifically, the present inventors have found that a cardiac infarction model which has a reduced animal mortality and is stable can be generated by ligating a blood vessel downstream of an arterial site to be occluded and subsequently subjecting it to complete occlusion of the artery at the site to be occluded.

Moreover, the present inventors have found that by employing an angiographic apparatus in delivering an autologous blood clot coagulated with a coagulating agent into an artery to form an embolus, an artery can be selectively occluded with the blood clot at a desired site within an animal, thereby generating an arterial embolus animal model in which the above-described problems have been solved.

Therefore, the present invention provides:
(1) a method for generating a non-human model animal of an arterial occlusive disease, wherein the method is characterized in that an artery is occluded at a specific site by ligating it, or with an autologous blood clot;
(2) a method according to (1), wherein the method is characterized in that in a non-human experimental animal, a blood vessel is ligated downstream of an arterial site of the heart to be occluded; followed by occluding the artery at said site, and wherein the method is for generating a cardiac infarction animal model;
(3) a method according to (2), wherein the experimental animal is a pig;
(4) a method according to (2) or (3), wherein the ligating is performed using a suture or clip and the occluding is carried out using an ameroid ring;
(5) a kit for generating a cardiac infarction animal model, wherein the kit comprises, as essential components, means for occluding an artery and means for ligating an artery and is employed in a method of any one of (2) to (4); and
(6) a cardiac infarction animal model which is generated by a method according to any one of (2) to (4) or a kit according to (5).

The present invention further provides:
(7) a method according to (1), wherein the method is characterized by adding an coagulating agent to autologous blood obtained from a non-human animal to form an autologous blood clot and subsequently delivering the autologous blood clot to a lumen of an artery of interest, and further characterized by employing angiographic apparatus in delivering the autologous blood clot to the arterial lumen, and wherein the method is for generating an arterial embolus animal model;
(8) a method according to (7), wherein the autologous blood clot is in a long-axis form;
(9) a method according to (7) or (8), wherein the angiographic apparatus is an X-ray angiographic apparatus; (10) a method according to any one of (7) to (9), wherein the method is further characterized by immersing the autologous blood clot in a solution of an iodine-containing agent, whereby the blood clot can be visualized by means of angiographic apparatus;
(11) a method according to any one of (7) to (10), wherein the artery is a cerebral or cardiac artery;
(12) a method according to any one of claims (7) to (11), wherein the method is further characterized by observing the state of the bloodstream using positron emission tomography (PET);
(13) a system for generating an arterial embolus animal model, wherein the system comprises, as essential components, means for forming an autologous blood clot with a coagulating agent, means for delivering the autologous blood clot into an artery, and an angiographic apparatus, and wherein the kit is used in a method according to any one of (7) to (12);
(14) a system according to (13), further comprising a PET apparatus; and
(15) a cardiac infarction animal model which is obtained employing a method according to any one of (7) to (12) or a system of (13) or (14).

According to the present invention, there are provided methods and kits for generating cardiac infarction animals, which allow complete occluding of trunk arteries, have a low animal mortality, and provide stable pathological conditions. In addition, according to the present invention, there are provided methods for generating vascular embolus animal models, which have a high certainty of vascular embolus and a small variation in its degree and are capable of quantitative analysis. Employing the methods of the present invention will diminish invasiveness to animals and also reduce animal damage.

### BRIEF DESCRIPTION OF THE DRAWINGS

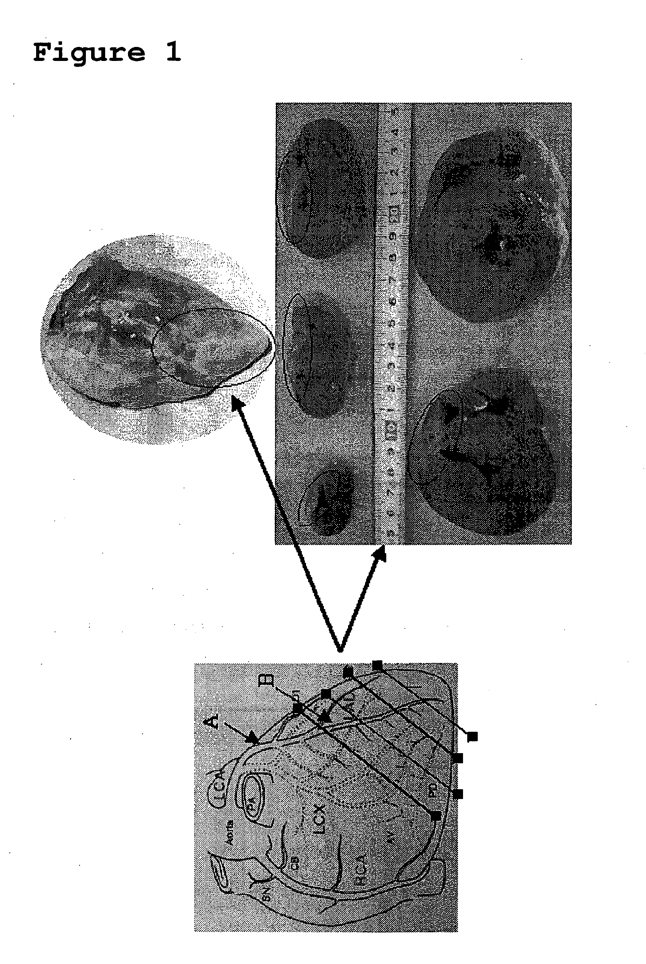

Fig. 1 shows the site for treatment according to the present invention (left panel) and is photographs showing the condition of infarction of the cardiac muscle of an individual (right panel). Arrow A in the left panel indicates the site at which an ameroid ring was applied and arrow B indicates the ligating site, and sections were cut along planes perpendicular to the plane comprising the oblique lines. There can be seen fibrosis in the area indicated by the circle in the right panel. The right upper panel is a photograph showing the appearance of the heart after the treatment and the right lower panel is a photograph showing heart sections at 1-cm intervals.
Fig. 2 is graphs showing the number (upper panel) and the rate (lower panel) of surviving animals in a period up to one month after the treatment with respect to the method of the present invention and a conventional method.
Fig. 3 represents photographs showing the comparison of blood clots at 24 hours after blood collecting (left panels, upper: control, middle: ADP addition, lower: thrombin addition), and a usual photograph (right upper panel) and X-ray photograph (right lower panel) of blood clots immersed into a povidone-iodine solution.
Fig. 4 represents cerebrovascular X-ray images before and immediately after occluding the left middle cerebral artery with an autologous blood clot (left and right panel, respectively) .
Fig. 5 represents PET images showing cerebral bloodstream (upper), rate of oxygen uptake (middle), and oxygen metabolism (lower) over time after the occlusion, which are images taken, from left to right, at 15, 30, 60, 120, 180 minutes after the occlusion.

### PREFERRED MODE FOR CARRYING OUT THE INVENTION

In one aspect, the present invention provides a method for generating a non-human model animal of an arterial occlusive disease, wherein the method is characterized in that an artery is occluded at a specific site by ligating it, or with an autologous blood clot.

In a specific embodiment of the aspect described above, the present invention provides a method for generating a cardiac infarction animal model, wherein the method is characterized in that in a non-human experimental animal, a blood vessel is ligated downstream of an arterial site of the heart to be occluded, followed by occluding the artery at the site to be occluded.

As animals for use in the present invention, any species for research will be applicable, if the animals are experimental animals (which, of course, exclude humans), and such experimental animals can be selected as appropriate. Examples of experimental animals include pigs, rats, mice, rabbits, guinea pigs, dogs, cats, monkeys, cows, horses, sheep and others. Among them, pigs, which have been commonly used in recent years and in particular, do not have many collateral vessels, are useful for the present invention. Pigs resemble humans in many aspects in terms of (1) physiological and anatomical characteristics; (2) physiology on digestion and absorption due to feeding habit, and (3) properties, such as distribution of coronary artery, endothelial structure of artery, and others, and are an animal suitable for the present invention also from the viewpoint of recent concern about animal welfare. Miniature pigs, in particular, have a well-defined genetic background and are particularly suitable for the present invention.

An organ to which the method and kit of the present invention is to be applied is the heart.

In the specification, "occlusion" means that the percentage of occlusion is usually about 80% or higher and preferably about 90% or higher, as measured by the method described in the Examples.

An arterial site to be occluded can be any site and selected as appropriate, depending upon factors, such as the location, size, and degree of a lesion needed, and kind of an animal used. Typically, an arterial site to be occluded is along the anterior descending branch just downstream of the branch of the left coronary artery (arteria coronaria sinistra).

The method of the present invention for generating a cardiac infarction model involves two steps. First, a means for ligating an artery is employed to ligate an artery downstream of an arterial site of the heart to be occluded. It is desirable to achieve complete ligation. It is important that by carrying out this local ligation, tolerance to ischemia is imparted to the whole cardiac muscle. As a means for ligating an artery, sutures are typical, although other means can be used, for example, clips and the like.
For clips, metallic small-sized clips are preferred. The ligation site is any site, if the site is distal along the artery to be occluded and neither fibrillates nor brings about animal death, even in the case of complete ligation. A site just downstream from the second branch of the anterior descending branch is preferred. The ligation is followed by occluding the arterial site to be occluded, after a given period of time, employing a means for occluding an artery. That is, occlusion is carried out after the tolerance to ischemia has been established in the whole cardiac muscle. Generally, treatments for occluding the artery to be occluded are carried out, as a guide, when about 30 minutes has passed after the ligation. When an ameroid ring is employed for occlusion in the method of the present invention, as described below, it will take about two weeks to accomplish complete occlusion after the ligation. The percentage of occlusion at 24 hours after the occluding treatment will reach approximately 60-70%, depending upon the wall thickness.

Next, at the arterial site to be occluded, the arterial site to be occluded is occluded employing a means for occluding an artery. During that period, it is preferable to prevent vascular spasm, for example, by applying a xylocaine jelly onto the artery. As means for occluding an artery, ameroid rings are typical, although any other means can be used, if it allows gradual constricting of a blood vessel over time in a similar manner, so that occlusion takes place. After occluding the artery, the thoracic cavity is closed and the procedures are completed. Closing of the thoracic cavity can be done according to routine procedures.

Additionally, the present invention provides a kit for generating a cardiac infarction model, wherein the kit is employed for the above-mentioned method for generating a cardiac infarction model. The kit comprises, as essential components, a means for occluding an artery and a means for ligating an artery. The kit is generally accompanied with instructions for carrying out the method of the present invention for generating a cardiac infarction model.

Animal models of cardiac infarction which can be obtained by the method or kit of the present invention possess stable pathological conditions and are very useful, for example, in medical studies of the circulatory system, on organ transplantation, or on regenerative medicine, or alternatively in the development of medicaments for circulatory system diseases.

In a further embodiment of the aspect described above, the present invention provides a method for generating a non-human animal model of arterial embolus, wherein the method is characterized in that an autologous blood clot obtained by adding a coagulating agent is delivered into an artery to form an embolus and an angiographic apparatus is employed in delivering the autologous blood clot.

Animals for use in the method of the present invention can be any animals excluding humans. Preferable animals include, for example, rodent animals (rats, mice, and others), primates (rhesus monkeys, chimpanzees, and others), dogs, cats, pigs and others.

The present invention is applicable to any artery of an animal. Preferably, the present invention is applied to a cerebral or cardiac artery.

The method according to the present invention involves two steps. The first step is for preparing an autologous blood clot. Blood is drawn beforehand from the animal of interest and mixed with a coagulating agent, and the mixture is subsequently incubated for a predetermined period of time, so as to obtain an embolus in a sufficiently solidified state. Drawing of blood from an animal can be done according to routine procedures. In the case of rodent animals, for example, blood can be drawn from the tail artery. The amount of blood to be drawn can be selected as appropriate, depending upon the kind of animal, the purpose of the treatments, etc.

It is preferable that the morphology of the autologous blood clots to be delivered is in a long-axis shape with respect to a blood vessel. By forming a long-axis shape, an autologous blood clot will take a shape compatible with the shape and running of a blood vessel and achieve easy flow into peripheral blood vessels, thereby allowing one to produce an embolus in a wide vascular area. In order to obtain an autologous blood clot of a long-axis shape, for example, in mixing an autologous blood and a coagulating agent, followed by incubation, the mixture is filled into and incubated in a container, for example, an appropriate tube, which has a smaller inner diameter than that of a catheter to be used for the treatment. The incubating container can be of materials which do not have any adverse effect on blood coagulation, such as polyethylene. Incubation times and temperatures should be selected so as to achieve sufficient solidification and can be modified as appropriate, depending upon the kind of animals, the condition of the autologous blood, the properties of a desired embolus, the type and site of the artery where the embolus is to be generated, the degree of the desired disease, etc. In general, incubation is carried out at a temperature near the body temperature of an animal of interest for about one day. These conditions and devices for incubation are those which can be readily modified and selected by those skilled in the art. Here, a coagulating agent refers to an agent capable of coagulating an autologous blood to an extent enough to cause a desired embolus. A variety of coagulating agents are known, including, for example, ADP (adenosine diphosphate) which has an effect of platelet aggregation, thrombin which promotes fibrin and embolus formation, and others. The class and concentration of coagulating agents can be selected as appropriate by those skilled in the art, depending upon the purpose. For example, autologous blood can be coagulated by holding the blood at 37°C for 24 hours in a lukewarm bath or incubator with 30 or 100 µg/ml of ADP and 0.5 or 10% (w/v) of thrombin.

The second step of the method of the present invention is a step by which the autologous blood clot prepared as described above is delivered to a desired site of a blood vessel to form an embolus. The means for delivering an autologous blood clot is not limited in particular, although catheters are commonly employed. Routinely, a catheter which is compatible with the inner diameter of an artery where an embolus is to be formed can be used and inserted through a placed sheath, so that a catheter is placed in a blood vessel of interest. Catheters of various types are known, and one can select and use a catheter as appropriate, with a catheter for angiography being preferred. The sheath is generally placed in the femoral artery. In the placement of a catheter, it is important that the catheter is correctly guided to a desired site by employing an angiographic apparatus. Defining precisely the embolus site in this way allows one to make selective and quantitative evaluation of pathological conditions in the resulting models. A variety of angiographic apparatus are employed at present and one can select an appropriate apparatus, depending upon the animal used, purpose of use, etc. X-ray angiographic apparatus are commonly used and among them, apparatus having high resolution are preferred. For X-ray angiographic apparatus, contrast agents of various types are known, including ionic and non-ionic, which can be selected and used as appropriate. After guiding the catheter to the targeted site where an embolus is formed, the autologous blood clot prepared as described above is run through the catheter. In doing so, it is possible to eject the embolus contained in a tube, for example, by connecting a needle and a syringe filled with saline directly to a narrow tube having the embolus solidified therein and pushing the saline out from the syringe. Also, it is possible to smoothly introduce the embolus, for example, by placing in advance a tip of a tube in the inlet of a catheter. One can determine whether the embolus has occluded the artery of interest by means of a cerebral angiographic apparatus, for example, by an X-ray cerebral angiography using a contrast agent. It is also possible to identify the existence of the embolus during an X-ray cerebral angiography by immersing in advance the prepared embolus into a solution containing an iodine-containing agent.

In addition, one can investigate the condition of ischemia after the treatment, such as the distribution and degree of ischemia, by using PET to determine the bloodstream conditions, such as blood flow the and capability of oxygen uptake and oxygen metabolism, since it is not clear that ischemia has been actually generated by occlusion, in the case of only occluding a blood vessel with the above-described steps. A variety of PET apparatus are employed at present and one can select and use an appropriate apparatus. Measurement parameters for identification of ischemia can be selected as appropriate by those skilled in the art and measuring methods using PET are also known.

The present invention further provides a system for generating a non-human animal model of arterial embolus, comprising, as essential components, a means for forming an autologous blood clot with a coagulating agent, a means for delivering an autologous blood clot into an artery, and an angiographic apparatus. The system is employed in the method of the present invention for generating an arterial embolus animal model. The means for forming an autologous blood clot with a coagulating agent may involve, for example, a coagulating agent and an incubating vessel. The means for delivering an autologous blood clot into an artery may involve, for example, an angiographic catheter. The angiographic apparatus may be a high-resolution X-ray angiographic apparatus. The system may further involve, for example, a PET apparatus for determining the bloodstream condition after the treatment.

Arterial embolus animal models which can be generated by the method or system of the present invention have a small variation in the degree of vascular embolus and thus are capable of quantitative analysis. Arterial embolus animal models obtained according to the present invention, therefore, are very useful, for example, in methods for treatment or prevention of diseases and disorders caused by arterial embolus, development of new medicaments against these diseases, or studies on regenerative medicine.

The present invention is now further described specifically with reference to the following examples, and should not be construed to be limited thereto.

### EXAMPLES

Example 1: Generation of a pig model of chronic cardiac infarction
Male pigs weighing 20-25 kg were used. After anesthesia, pigs were fixed in the recumbent position with the left chest up and an anticoagulant, heparin, was administered at 100 IU/kg, followed by thoracotomy by dissection between the third and fourth ribs. Subsequently, portion beyond the second branch of the anterior descending branch was exposed and completely ligated with a suture (see arrow B of the left panel in Fig. 1). (Complete ligating at this site does not give rise to fibrillation, so not leading to death. This local ischemic treatment results in the tolerance to ischemia in the entire cardiac muscle.) After that, a portion of the anterior descending branch proximate to the bifurcation of the circumflex branch and the anterior descending branch was exposed and a xylocaine jelly was applied to the blood vessel to prevent vascular spasm associated with the treatment, followed by placement of an ameroid ring (also known as Ameroid Constrictor, RESEARCH INSTRUMENTS, SW, Inc.; I.D. 2.5 mm) (see arrow A of the left panel in Fig. 1) The thoracic cavity was closed as usual and the procedures were completed. As a control treatment, conventional procedures without prior complete ligating (i.e., by only occluding with an ameroid ring) were employed to apply the occluding procedures to the same arterial site in a manner similar to that described above.

At three months after the treatment according to the present invention as described above, the condition of animal hearts was examined. Significant fibrosis was observed in the apex cordis area of individual hearts and also inside the cardiac muscle of heart sections cut at intervals of 1 cm (Fig. 1, right panel). This fibrosis is an indication of the occurrence of definite occlusion. In the treatment according to the present invention, similar occlusion was observed with the cardiac muscle of all of the five pigs, indicating the stability and certainty of the method of the present invention. Each animal that was treated according to the present invention had a small variation in the percent occlusion, which was more than 90% for respective animals. In contrast, animals receiving the control treatment resulted in generation of unstable and uncertain occlusion and occlusion, as seen in the right panel of Fig. 1, was observed only in the cardiac muscle of one of the five pigs. Each animal receiving the control treatment had a wide variation in the percent occlusion, which was 69.8-100%. The percent occlusion was calculated according the following equation: [(VASCULAR LUMEN AREA IN FRONT OF RING PLACEMENT SITE - VASCULAR LUMEN AREA AT RING PLACEMENT SITE)/(VASCULAR LUMEN AREA IN FRONT OF RING PLACEMENT SITE)] × 100 (%), by measuring the vascular lumen area in front of and at the ring ameroid placement site at three months after the treatment.

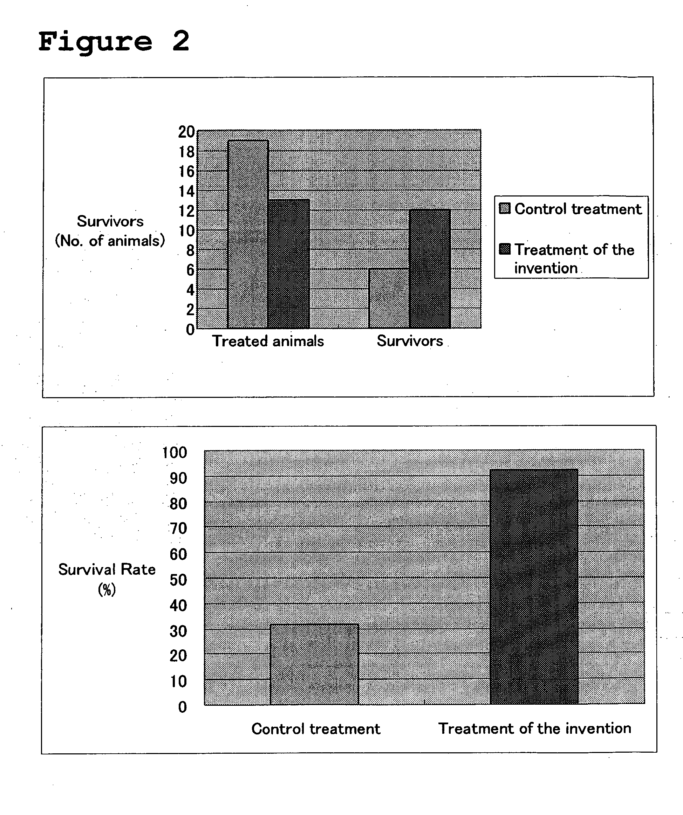

The treatment according to the present invention and the control treatment were conducted on more pigs in the same way as described above to examine the survival rate after the treatment. When the treatment according to the present invention was carried out, 12 out of 13 animals survived until one month after the treatment, i.e., the survival rate exceeded 90% (Fig. 2). When the control treatment was carried out, only 6 out of 19 animals survived until one month after the treatment, i.e., the survival rate, which was about 30%, was low (Fig. 2).

Thus, it appears that according to the present invention, disadvantages of conventional ligation procedures and methods in which only occlusion with ameroid rings is used, such as wide variation of occlusion, high mortality rates, and unstable pathological conditions, can be eliminated.

### Example 2: Studies on coagulating agents, conditions, and others

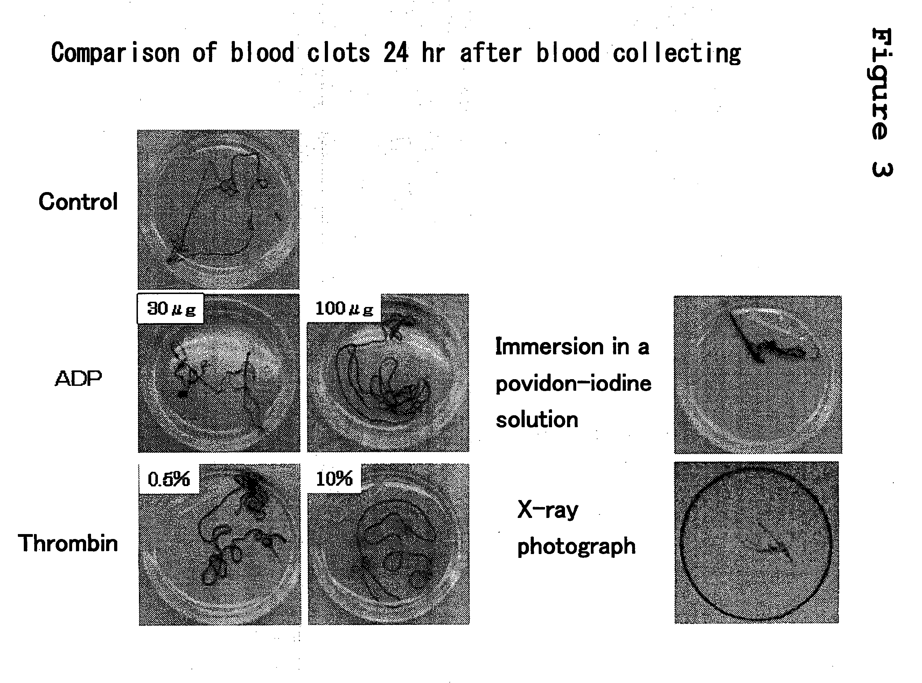

As coagulating agents, ADP and thrombin were used to examine the degree of coagulation. ADP was used at concentrations of 30 and 100 µg/ml (without thrombin at both concentrations), and thrombin at concentrations of 0.5 and 10% (w/v) (without ADP at both concentrations). Blood was drawn from the ventral tail artery of a crab-eating macaque, was placed into a catheter with or without adding a coagulating agent, incubated for 24 hours at 37°C, and then pushed out to observe the condition of coagulation. As control, a sample was used to which neither of the coagulating agents was added (blood which was coagulated alone). The results are shown in the left panel of Fig. 3. It was found that the higher the concentration of ADP, the better coagulation took place. Thrombin resulted in comparable coagulation at both 0.5 and 10% (w/v). Next, a blood clot which was obtained by incubating blood for 24 hours at 37°C with 100 µg of ADP and 10% (w/v) of thrombin was immersed in a povidone-iodine solution, followed by X-ray photography. As shown in the right panel of Fig. 3, there was observed a shade corresponding to the blood clot, allowing one to identify the location of a blood clot by means of an X-ray photographic apparatus.

### Example 3: Generation of an acute cerebral trunk arterial embolus model

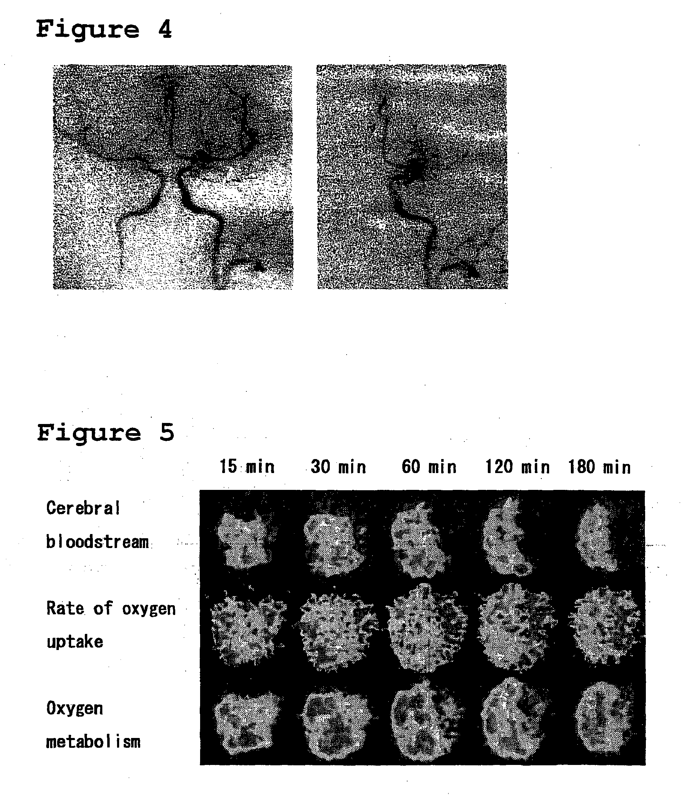

As a study animal a male crab-eating macaque weighing about 6 kg was utilized. A sufficient amount of blood (1 ml) was drawn from the ventral tail artery of the animal with a syringe containing in advance ADP (a final concentration of 100 µg/ml) and thrombin (a final concentration of 10% (w/v)) as coagulants, and filled immediately into a polyethylene tube (O.D.: 0.965 mm, I.D.: 0.58 mm). An embolus having a well-solidified state was formed by leaving the mixture of blood and coagulants at 37°C for 24 hours while keeping the mixture in the tube. Subsequently, a very fine catheter for cerebral angiography which was in conformity to an arterial diameter (3 French) was used and inserted through a sheath placed into the femoral artery, so as to place the catheter in a targeted cerebral vessel (left middle cerebral artery). During this, the catheter was guided using a high-resolution X-ray cerebral angiographic apparatus (Mobile C-Arm SERIES 9800^{™}, manufactured by GE Yokogawa Medical Systems) with a contrast agent (Omnipaque 350). After guiding the catheter to the targeted site, the autologous blood clot prepared as described above was run through the catheter. In doing so, the embolus within the tube was ejected by connecting a needle and a syringe filled with saline directly to a narrow tube having the embolus solidified therein and pushing the saline out through the syringe. It was possible to introduce the embolus smoothly by placing the tip of the narrow tube in advance in the inlet of the catheter. Whether the embolus had occluded the artery of interest was determined by means of X-ray cerebral angiography using the contrast agent. Fig. 4 shows the result obtained when the middle cerebral artery was occluded. It can be seen that blood did not flow in the blood vessel downstream from the occluded site which is indicated by the arrow (Fig. 4, right panel). In addition, the cerebral bloodstream, rate of oxygen uptake, and oxygen metabolism were measured with PET (model: ECAT EXACT 47, manufactured by Siemens) to determine the distribution and degree of ischemia (Fig. 5), since it was unknown that cerebral ischemia actually took place in the case of only occluding the cerebral vessel. Although a reduction in cerebral bloodstream was observed in correspondence with the left middle cerebral artery area, the oxygen metabolism tended to be maintained, but to a small extent, and so it was found to be in a severe ischemic condition.

As mentioned above, the present invention can generate desired conditions of ischemia by selectively occluding a blood vessel at a desired site with certainty. The present invention also allows for quantitively analysing and identifying ischemic conditions thus generated. In addition, the present invention brings the advantage of causing small damage due to low invasiveness to study animals.

### INDUSTRIAL APPLICABILITY

The methods and kits of the present invention for generating cardiac infarction models, as well as cardiac infarction animal models generated by the methods and kits can be employed in studies on arterial occlusive diseases, medical studies on organ transplantation and others, or alternatively in the development of medicaments for arterial occlusive diseases, and additionally in studies on regenerative medicine.

## Claims

1. A method for generating a non-human animal model of an arterial occlusive disease, wherein the method is **characterized in that** an artery is occluded at a specific site by ligating it, or with an autologous blood clot.

2. A method according to claim 1, wherein the method is **characterized in that** in a non-human experimental animal, a blood vessel is ligated downstream of an arterial site of the heart to be occluded, followed by occluding the artery at said site, and wherein the method is for generating a cardiac infarction animal model.

3. A method according to claim 2, wherein the experimental animal is a pig.

4. A method according to claim 2 or 3, wherein the ligating is performed using a suture or clip and the occluding is carried out using an ameroid ring.

5. A kit for generating a cardiac infarction animal model, wherein the kit comprises, as essential components, means for occluding an artery and means for ligating an artery and is employed in a method of any one of claims 2 to 4.

6. A cardiac infarction animal model which is generated by a method according to any one of claims 2 to 4 or a kit according to claim 5.

7. A method according to claim 1, wherein the method is **characterized by** adding coagulating agent to autologous blood obtained from a non-human animal to form an autologous blood clot and subsequently delivering the autologous blood clot to a lumen of an artery of interest, and further **characterized by** employing angiographic apparatus in delivering the autologous blood clot to the arterial lumen, and wherein the method is for generating an arterial embolus animal model.

8. A method according to claim 7, wherein the autologous blood clot is in a long-axis form.

9. A method according to claim 7 or 8, wherein the angiographic apparatus is an X-ray angiographic apparatus.

10. A method according to any one of claims 7 to 9, wherein the method is further **characterized by** immersing the autologous blood clot in a solution of an iodine-containing agent, whereby the blood clot can be visualized by means of angiographic apparatus.

11. A method according to any one of claims 7 to 10, wherein the artery is a cerebral or cardiac artery.

12. A method according to any one of claims 7 to 11, wherein the method is further **characterized by** observing the state of the bloodstream using positron emission tomography (PET).

13. A system for generating an arterial embolus animal model wherein the system comprises, as essential components, means for forming an autologous blood clot with a coagulating agent, means for delivering the autologous blood clot into an artery, and an angiographic apparatus, and wherein the kit is used in a method according to any one of claims 7 to 12.

14. A system according to claim 13, further comprising a PET apparatus.

15. A cardiac infarction animal model which is obtained employing a method according to any one of claims 7 to 12 or a system of claim 13 or 14.
